# EUROPEAN PATENT SPECIFICATION

(11) **EP 3 053 528 B1**
(45) Date of publication and mention of the grant of the patent: **29.08.2018**
(21) Application number: 15202807.2
(22) Date of filing: 28.12.2015
(51) Int. Cl.: A61B 8/08, A61B 8/00, G01S 7/52

(54) **ULTRASOUND DIAGNOSIS APPARATUS AND OPERATING METHOD THEREOF**
ULTRASCHALLDIAGNOSEVORRICHTUNG UND BETRIEBSVERFAHREN DAVON
APPAREIL DE DIAGNOSTIC ULTRASONIQUE ET PROCÉDÉ DE FONCTIONNEMENT ASSOCIÉ

(30) Priority: 05.02.2015 KR 20150018096
(43) Date of publication of application: 10.08.2016
(60) Divisional application: 18181565.5
(73) Proprietor: Samsung Medison Co., Ltd., Hongcheon-gun, Gangwon-do, 25108 (KR)
(72) Inventor: Lee, Jae-sung, Gangwon-do (KR)
(74) Representative: Frey, Sven Holger

(56) References cited:
- US-A1- 2005 096 538
- US-A1- 2014 187 946
- US-B2- 6 589 176

## Description

### RELATED APPLICATIONS

This application claims the benefit of Korean Patent Application No. 10-2015-0018096, filed on February 5, 2015, in the Korean Intellectual Property Office, the disclosure of which is incorporated herein in its entirety by reference.

### BACKGROUND

### 1. Field

One or more embodiments of the present inventive concept relate to an ultrasound diagnosis apparatus which may change a condition for transceiving an ultrasound beam based on the position of an object of interest from a plurality of pieces of ultrasound data, an ultrasound diagnosis method using the ultrasound diagnosis apparatus according thereto, and a computer-readable recording medium having recorded thereon a program for executing the ultrasound diagnosis method.

### 2. Description of the Related Art

Ultrasound diagnosis apparatuses transmit ultrasound signals generated by transducers of a probe to an object and receive echo signals reflected from the object, thereby acquiring at least one image of an internal part of the object (e.g., soft tissue or blood flow). In particular, ultrasound diagnosis apparatuses are used for medical purposes including observation of the interior of an object, detection of foreign substances, and diagnosis of damage to the object. Such ultrasound diagnosis apparatuses provide high stability, display images in real time, and are safe due to no radioactive exposure, compared to X-ray apparatuses. Therefore, ultrasound diagnosis apparatuses are widely used together with other image diagnosis apparatuses including a computed tomography (CT) apparatus, a magnetic resonance imaging (MRI) apparatus, and the like.

An ultrasound diagnosis apparatus transmits ultrasound waves to a fixed position or receives ultrasound waves from the fixed position. Accordingly, an ultrasound image of an object of interest may be acquired only when the object of interest is located at the fixed position. Also, deviation in the time for acquiring an ultrasound image, the reliability of an ultrasound image, or the quality of an ultrasound image varies greatly depending on the proficiency of a user. Also, even when the object of interest is well displayed on an ultrasound image, the position of the object of interest is changed on the ultrasound image according to a movement of the object of interest or a probe. Accordingly, the user may have difficulty performing diagnosis based on the ultrasound image.

Also, according to the ultrasound diagnosis apparatus, when the user acquires an ultrasound image of the object of interest for a long time, lots of time and effort are taken to acquire the ultrasound image of the same object of interest. Also, according to the ultrasound diagnosis apparatus, it is difficult to acquire an ultrasound image at a particular angle with respect to the object of interest.

The US 2005/096538 A1 discloses a medical imaging system automatically acquiring two-dimensional images representing a user-defined region of interest despite motion. The plane of acquisition is updated or altered adaptively as a function of detected motion. The user-designated region of interest is then continually scanned due to the alteration in scan plane position. A multidimensional array is used to stabilize imaging of a region of interest in a three-dimensional volume. The user defines a region of interest for two-dimensional imaging. Motion is then detected. The position of a scan plane used to generate a subsequent two-dimensional image is then oriented as a function of the detected motion within the three-dimensional volume. By repeating the motion determination and adaptive alteration of the scan plane position, real time imaging of a same region of interest is provided while minimizing the region of interest fading into or out of the sequence of images.

The US 2014/187946 A1 discloses a computer-implemented method for active control of ultrasound image acquisition including accessing image data representing a series of ultrasound images acquired over a period of time from an ultrasound probe and identifying an object of interest in at least one of the images. The method further includes detecting changes in a position of the ultrasound probe and/or an orientation of the ultrasound probe over the period of time with respect to the object of interest, or changes in a position of the object of interest and/or an orientation of the object of interest over the period of time with respect to the ultrasound probe. The method further includes adjusting at least one ultrasound image acquisition parameter based on the detected changes in the position and/or the orientation of the ultrasound probe and/or based on the detected changes in the position and/or the orientation of the object of interest.

In the US 6 589 176 B2 an ultrasonic diagnostic imaging system is provided in which anatomical images are stabilized in the presence of probe motion, anatomical motion, or both. A decision processor analyzes motional effects and determines whether to inhibit or allow image stabilization. Images are anatomically aligned on the basis of probe motion sensing, image analysis, or both. The stabilization system can be activated either manually or automatically and adaptively.

### SUMMARY

One or more embodiments of the present inventive concept include an ultrasound diagnosis apparatus which may obtain the position of an object of interest from a plurality of pieces of ultrasound data and change a condition for transceiving an ultrasound beam based on the position of the object of interest, an ultrasound diagnosis method using the ultrasound diagnosis apparatus according thereto, and a computer-readable recording medium having recorded thereon a program for executing the ultrasound diagnosis method.

According to the present exemplary embodiments, deviation in the diagnosis according to a user's measurement ability may be reduced. Also, difficulty occurring during using an ultrasound diagnosis apparatus, such as disappearance of an object of interest in an ultrasound image due to a movement of the object of interest or a probe, may be reduced.

Also, when a user acquires an ultrasound image of the object of interest for a long time, the ultrasound image of the same object of interest may be easily acquired. Furthermore, an ultrasound image at a particular angle with respect to an object of interest may be easily acquired.

Additional aspects will be set forth in part in the description which follows and, in part, will be apparent from the description, or may be learned by practice of the presented embodiments.

According to one or more embodiments of the present inventive concept, an ultrasound diagnosis apparatus according to claim 1 includes a data acquirer acquiring first ultrasound data and second ultrasound data with respect to an object, and a controller detecting a first position of an object of interest included in the object on the first ultrasound data, detecting a second position of the object of interest based on the first position, and changing a condition for transceiving an ultrasound beam based on the second position.

The controller may detect the second position of the object of interest on the second ultrasound data based on a degree of correlation between at least one of pixel values and voxel values of the object of interest on the first ultrasound data and at least one of pixel values and voxel values on the second ultrasound data.

The controller may change the condition for transceiving an ultrasound beam further based on the first position.

The controller may acquire a first coordinate value indicating the first position on the first ultrasound data, acquire a second coordinate value indicating the second position on the second ultrasound data, and change the condition for transceiving an ultrasound beam based on a difference value between the first coordinate value and the second coordinate value.

The controller may acquire a coordinate value of a center point of the object of interest on the first ultrasound data, as the first coordinate value, and a coordinate value of a center point of the object of interest on the second ultrasound data, as the second coordinate value.

The controller may change the condition for transceiving an ultrasound beam based on a difference value between the second position and a preset position.

The ultrasound diagnosis apparatus may further include an input unit receiving a user's input for setting a region of interest (ROI) on a first ultrasound image based on the first ultrasound data, wherein the controller detects the first position of the object of interest in the ROI.

The condition for transceiving an ultrasound beam may include at least one of a receiving depth of an ultrasound beam, a width of an ultrasound beam, a steering angle of an ultrasound beam, and a focusing position of an ultrasound beam.

The controller may change in real time a condition for an ultrasound beam transmitted toward the object.

The ultrasound diagnosis apparatus may further include a display displaying a second ultrasound image including the object of interest based on the second ultrasound data, and displaying the second ultrasound image by changing at least one of a shape, a size, and a position of the first ultrasound image according to the change of the condition for transceiving an ultrasound beam.

According to one or more embodiments of the present inventive concept, a method of operating an ultrasound diagnosis apparatus according to claim 11 includes acquiring first ultrasound data with respect to an object including an object of interest, detecting a first position of the object of interest on the first ultrasound data, acquiring second ultrasound data with respect to the object, detecting a second position of the object of interest based on the first position on the second ultrasound data, and changing a condition for an ultrasound beam transmitted toward the object based on the second position.

The detecting of the second position may be based on a degree of correlation between at least one of pixel values and voxel values of the object of interest on the first ultrasound data and at least one of pixel values and voxel values on the second ultrasound data.

In the changing of the transceiving condition, the condition for transceiving an ultrasound beam may be changed further based on the first position.

The detecting of the first position may include acquiring a first coordinate value indicating the first position on the first ultrasound data, the detecting of the second position comprises acquiring a second coordinate value indicating the second position on the second ultrasound data, and the changing of the transceiving condition comprises changing the condition for transceiving an ultrasound beam based on a difference value between the first coordinate value and the second coordinate value.

The detecting of the first position may include acquiring a coordinate value of a center point of the object of interest on the first ultrasound data, as the first coordinate value, and the detecting of the second position comprises a coordinate value of a center point of the object of interest on the second ultrasound data, as the second coordinate value.

The changing of the transceiving condition may include changing the condition for transceiving an ultrasound beam based on a difference value between the second position and a preset position.

The method may further include receiving a user's input for setting a region of interest (ROI) on a first ultrasound image based on the first ultrasound data, wherein the detecting of the first position comprises detecting the first position of the object of interest in the ROI.

The condition for transceiving an ultrasound beam may include at least one of a receiving depth of an ultrasound beam, a width of an ultrasound beam, a steering angle of an ultrasound beam, and a focusing position of an ultrasound beam.

A condition for an ultrasound beam transmitted toward the object may be changed in real time.

The method may further include displaying a second ultrasound image including the object of interest based on the second ultrasound data, and displaying the second ultrasound image by changing at least one of a shape, a size, and a position of the first ultrasound image according to the change of the condition for transceiving an ultrasound beam.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a structure of an ultrasound diagnosis apparatus according to an exemplary embodiment;
FIG. 2 is a block diagram illustrating a structure of a wireless probe according to an exemplary embodiment;
FIG. 3 is a block diagram illustrating a structure of an ultrasound diagnosis apparatus according to another exemplary embodiment;
FIGS. 4A and 4B illustrate a process of acquiring ultrasound data, according to an exemplary embodiment;
FIGS. 5A and 5B schematically illustrate ultrasound images acquired based on ultrasound data according to an exemplary embodiment;
FIG. 6 illustrates an operation of an ultrasound diagnosis apparatus, according to an exemplary embodiment;
FIGS. 7A and 7B schematically illustrate ultrasound images according to an exemplary embodiment;
FIGS. 8A and 8B illustrate a process of acquiring ultrasound data according to an exemplary embodiment;
FIGS. 9A and 9B schematically illustrate ultrasound images acquired based on ultrasound data according to an exemplary embodiment;
FIG. 10 illustrates first ultrasound data according to an exemplary embodiment;
FIG. 11 illustrates a process in which an ultrasound diagnosis apparatus according to an exemplary embodiment searches for a position of an object of interest in second ultrasound data;
FIG. 12 illustrates the second ultrasound data according to an exemplary embodiment;
FIG. 13 illustrates a first position and a second position on volume data according to an exemplary embodiment;
FIG. 14 illustrates the first position and the second position in space according to an exemplary embodiment;
FIG. 15 is a view for explaining a method of changing a transceiving condition of an ultrasound diagnosis apparatus, according to an exemplary embodiment;
FIGS. 16A and 16B are views for explaining a method of changing a transceiving condition of an ultrasound diagnosis apparatus, according to an exemplary embodiment; and
FIG. 17 is a flowchart for describing a method of operating an ultrasound diagnosis apparatus, according to an exemplary embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, the terms used in the specification will be briefly described, and then the present inventive concept will be described in detail.

The terms used in this specification are those general terms currently widely used in the art in consideration of functions regarding the present inventive concept, but the terms may vary according to the intention of those of ordinary skill in the art, precedents, or new technology in the art. Also, specified terms may be selected by the applicant, and in this case, the detailed meaning thereof will be described in the detailed description of the inventive concept. Thus, the terms used in the specification should be understood not as simple names but based on the meaning of the terms and the overall description of the inventive concept.

Throughout the specification, it will also be understood that when a component "includes" an element, unless there is another opposite description thereto, it should be understood that the component does not exclude another element and may further include another element. In addition, terms such as "... unit", "... module", or the like refer to units that perform at least one function or operation, and the units may be implemented as hardware or software or as a combination of hardware and software.

Throughout the specification, an "ultrasound image" refers to an image of an object, which is obtained using ultrasound waves. Furthermore, an "object" may be a human, an animal, or a part of a human or animal. For example, the object may be an organ (e.g., the liver, the heart, the womb, the brain, a breast, or the abdomen), a blood vessel, or a combination thereof. Furthermore, the object may be a phantom. The phantom means a material having a density, an effective atomic number, and a volume that are approximately the same as those of an organism. For example, the phantom may be a spherical phantom having properties similar to a human body.

In the present specification, an object and an object of interest are used separately. For example, an object may be an examinee, that is, the object may be a person or an animal. In contrast, an object of interest is included in the object and may be a part of a person or an animal where a user desires to acquire an ultrasound image.

Furthermore, throughout the specification, a "user" may be, but is not limited to, a medical expert, such as a medical doctor, a nurse, a medical laboratory technologist, a medical image expert, or a technician who repairs a medical apparatus.

Embodiments of the inventive concept now will be described more fully hereinafter with reference to the accompanying drawings, in which illustrative embodiments of the inventive concept are shown.

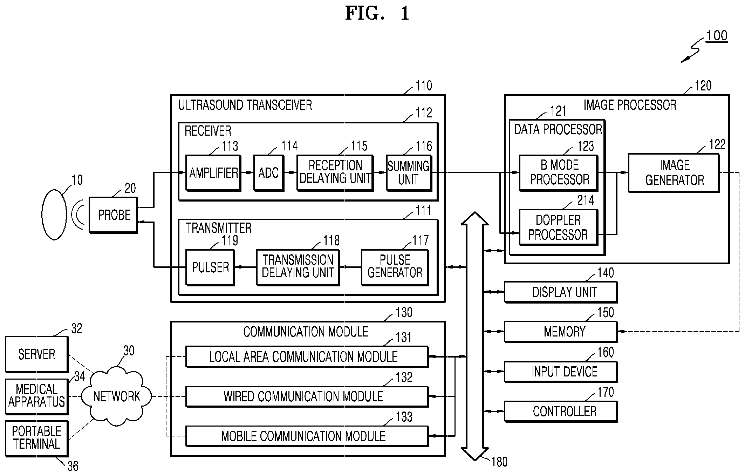

FIG. 1 is a block diagram showing a configuration of an ultrasound diagnosis apparatus 100 according to an embodiment of the present inventive concept. Referring to FIG. 1, the ultrasound diagnosis apparatus 100 may include a probe 20, an ultrasound transceiver 110, an image processor 120, a communication module 130, a display 140, a memory 150, an input device 160, and a controller 170, which may be connected to one another via buses 180.

The ultrasound diagnosis apparatus 100 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 transmits ultrasound waves to an object 10 in response to a driving signal applied by the ultrasound transceiver 110 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 100 by wire or wirelessly. According to embodiments of the present inventive concept, the ultrasound diagnosis apparatus 100 may include a plurality of probes 20.

A transmitter 111 supplies a driving signal to the probe 20. The transmitter 111 includes a pulse generator 117, a transmission delaying unit 118, and a pulser 119. The pulse generator 117 generates pulses for forming transmission ultrasound waves based on a predetermined pulse repetition frequency (PRF), and the transmission delaying unit 118 delays the pulses by delay times necessary for determining transmission directionality. The pulses which have been delayed correspond to a plurality of piezoelectric vibrators included in the probe 20, respectively. The pulser 119 applies a driving signal (or a driving pulse) to the probe 20 based on timing corresponding to each of the pulses which have been delayed.

A receiver 112 generates ultrasound data by processing echo signals received from the probe 20. The receiver 112 may include an amplifier 113, an analog-to-digital converter (ADC) 114, a reception delaying unit 115, and a summing unit 116. The amplifier 113 amplifies echo signals in each channel, and the ADC 114 performs analog-to-digital conversion with respect to the amplified echo signals. The reception delaying unit 115 delays digital echo signals output by the ADC 114 by delay times necessary for determining reception directionality, and the summing unit 116 generates ultrasound data by summing the echo signals processed by the reception delaying unit 115. Also, according to embodiments of the present inventive concept, the receiver 112 may not include the amplifier 113. In other words, if the sensitivity of the probe 20 or the capability of the ADC 114 to process bits is enhanced, the amplifier 113 may be omitted.

The image processor 120 generates an ultrasound image by scan-converting ultrasound data generated by the ultrasound transceiver 110 and displays the ultrasound image. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 123 extracts B mode components from ultrasound data and processes the B mode components. An image generator 122 may generate an ultrasound image indicating signal intensities as brightness based on the extracted B mode components.

Similarly, a Doppler processor 124 may extract Doppler components from ultrasound data, and the image generator 122 may generate a Doppler image indicating a movement of an object as colors or waveforms based on the extracted Doppler components.

According to an embodiment of the present inventive concept, the image generator 122 may generate a three-dimensional (3D) ultrasound image via volume-rendering with respect to volume data and may also generate an elasticity image by imaging deformation of the object 10 due to pressure. Furthermore, the image generator 122 may display various pieces of additional information in an ultrasound image by using text and graphics. In addition, the generated ultrasound image may be stored in the memory 150.

A display 140 displays the generated ultrasound image. The display 140 may display not only an ultrasound image, but also various pieces of information processed by the ultrasound diagnosis apparatus 100 on a screen image via a graphical user interface (GUI). In addition, the ultrasound diagnosis apparatus 100 may include two or more displays 140 according to embodiments of the present inventive concept.

The communication module 130 is connected to a network 30 by wire or wirelessly to communicate with an external device or a server. The communication module 130 may exchange data with a hospital server or another medical apparatus in a hospital, which is connected thereto via a PACS. Furthermore, the communication module 130 may perform data communication according to the digital imaging and communications in medicine (DICOM) standard.

The communication module 130 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 130 may receive information about a diagnosis history or medical treatment schedule of a patient from a server and utilizes the received information to diagnose the patient. Furthermore, the communication module 130 may perform data communication not only with a server or a medical apparatus in a hospital, but also with a portable terminal of a medical doctor or patient.

The communication module 130 is connected to the network 30 by wire or wirelessly to exchange data with a server 32, a medical apparatus 34, or a portable terminal 36. The communication module 130 may include one or more components for communication with external devices. For example, the communication module 130 may include a local area communication module 131, a wired communication module 132, and a mobile communication module 133.

The local area communication module 131 refers to a module for local area communication within a predetermined distance. Examples of local area communication techniques according to an embodiment of the present inventive concept may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 132 refers to a module for communication using electric signals or optical signals. Examples of wired communication techniques according to an embodiment of the present inventive concept may include communication via a twisted pair cable, a coaxial cable, an optical fiber cable, and an Ethernet cable.

The mobile communication module 133 transmits or receives wireless signals to or from at least one selected from a base station, an external terminal, and a server on a mobile communication network. The wireless signals may be voice call signals, video call signals, or various types of data for transmission and reception of text/multimedia messages.

The memory 150 stores various data processed by the ultrasound diagnosis apparatus 100. For example, the memory 150 may store medical data related to diagnosis of an object, such as ultrasound data and an ultrasound image that are input or output, and may also store algorithms or programs which are to be executed in the ultrasound diagnosis apparatus 100.

The memory 150 may be any of various storage media, e.g., a flash memory, a hard disk drive, EEPROM, etc. Furthermore, the ultrasound diagnosis apparatus 100 may utilize web storage or a cloud server that performs the storage function of the memory 150 online.

The input device 160 refers to a means via which a user inputs data for controlling the ultrasound diagnosis apparatus 100. The input device 160 may include hardware components, such as a keypad, a mouse, a touch pad, a touch screen, and a jog switch. However, embodiments of the present inventive concept are not limited thereto, and the input device 160 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

The controller 170 may control all operations of the ultrasound diagnosis apparatus 100. In other words, the controller 170 may control operations among the probe 20, the ultrasound transceiver 110, the image processor 120, the communication module 130, the display 140, the memory 150, and the input device 160 shown in FIG. 1.

All or some of the probe 20, the ultrasound transceiver 110, the image processor 120, the communication module 130, the display 140, the memory 150, the input device 160, and the controller 170 may be implemented as software modules. However, embodiments of the present inventive concept are not limited thereto, and some of the components stated above may be implemented as hardware modules. Furthermore, at least one selected from the ultrasound transceiver 110, the image processor 120, and the communication module 130 may be included in the controller 170. However, embodiments of the present inventive concept are not limited thereto.

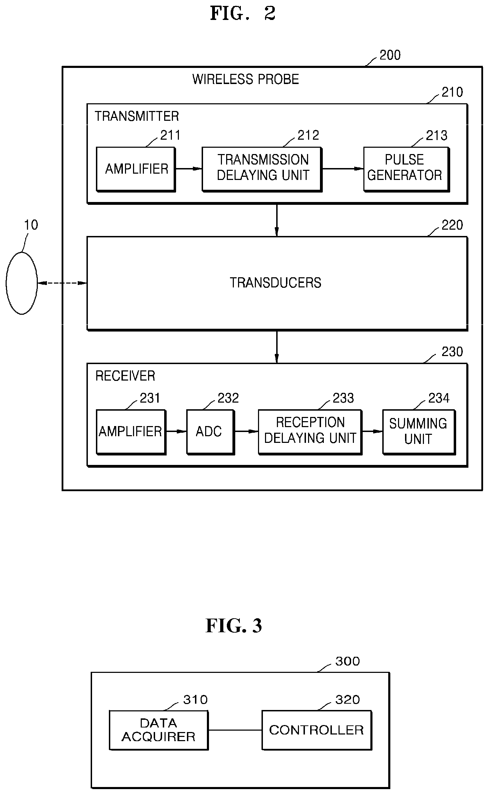

FIG. 2 is a block diagram showing a configuration of a wireless probe 200 according to an embodiment of the present inventive concept. As described above with reference to FIG. 1, the wireless probe 200 may include a plurality of transducers, and, according to embodiments of the present inventive concept, may include some or all of the components of the ultrasound transceiver 110 shown in FIG. 1.

The wireless probe 200 according to the embodiment shown in FIG. 2 includes a transmitter 210, a transducer 220, and a receiver 230. Since descriptions thereof are given above with reference to FIG. 1, detailed descriptions thereof will be omitted here. In addition, according to embodiments of the present inventive concept, the wireless probe 200 may selectively include a reception delaying unit 233 and a summing unit 234.

The wireless probe 200 may transmit ultrasound signals to the object 10, receive echo signals from the object 10, generate ultrasound data, and wirelessly transmit the ultrasound data to the ultrasound diagnosis apparatus 100 shown in FIG. 1.

An ultrasound diagnosis apparatus transmits ultrasound waves to a fixed position or receives ultrasound waves from the fixed position. Accordingly, an ultrasound image of an object of interest may be acquired only when the object of interest is located at the fixed position. Also, deviation in the time for acquiring an ultrasound image, the reliability of an ultrasound image, or the quality of an ultrasound image varies greatly according to proficiency of a user. Also, even when the object of interest is well displayed on an ultrasound image, the position of the object of interest is changed on the ultrasound image according to a movement of the object of interest or a probe. Accordingly, the user may have difficulty performing diagnosis based on the ultrasound image. Thus, an ultrasound diagnosis apparatus which may enable a user to acquire an ultrasound image more easily, and a method of operating an ultrasound diagnosis apparatus are demanded.

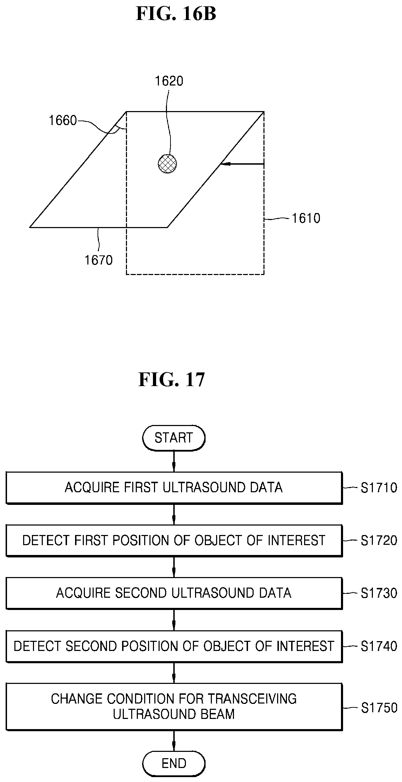

In the following description, an ultrasound diagnosis apparatus according to an exemplary embodiment, a method of operating an ultrasound diagnosis apparatus, and a computer-readable recording medium having recorded thereon a program for executing the method are described in detail with reference to FIGS. 3 and 17.

FIG. 3 is a block diagram illustrating a structure of an ultrasound diagnosis apparatus 300 according to another exemplary embodiment.

The ultrasound diagnosis apparatus 300 refers to all electronic apparatuses capable of receiving, processing, and/or outputting an ultrasound image, and may be used in medical imaging apparatuses such as an ultrasound imaging apparatus, a computed tomography (CT) apparatus, or a magnetic resonance imaging (MRI) apparatus. For example, the ultrasound diagnosis apparatus 300 may be included in a medical imaging apparatus.

Referring to FIG. 3, the ultrasound diagnosis apparatus 300 may include a data acquirer 310 and a controller 320.

The data acquirer 310 may acquire first ultrasound data and second ultrasound data about an object. Although the data acquirer 310 may acquire ultrasound data by scanning the object using an ultrasound signal, the present exemplary embodiment is not limited thereto. In an example, the data acquirer 310, which may correspond to the ultrasound transceiver 110 of FIG. 1, may receive an ultrasound echo signal transmitted by the probe 20 and acquire ultrasound data by using a received ultrasound echo signal.

In another example, the data acquirer 310 may receive scan information obtained as a scanning apparatus outside the ultrasound diagnosis apparatus 300 scans the object, for example, ultrasound data generated by converting the ultrasound echo signal, and acquire ultrasound data based on the scan information. In another example, the data acquirer 310 may receive ultrasound image data from an external apparatus through the network 30. However, the present exemplary embodiment is not limited thereto, and the ultrasound diagnosis apparatus 300 may acquire the ultrasound data in various methods.

The first ultrasound data and the second ultrasound data may be volume data that is three-dimensional data. The first ultrasound data and the second ultrasound data may include a plurality of voxels. A voxel value may include at least one of a luminance value and a color value of a corresponding voxel. The volume data may include a plurality of pieces of two-dimensional data.

Also, the first ultrasound data and the second ultrasound data may be plane data that is two-dimensional data. The first ultrasound data and the second ultrasound data may include a plurality of pixel values. A pixel value may include at least one of a luminance value and a color value of a corresponding pixel.

According to the present exemplary embodiment, the ultrasound diagnosis apparatus 300 may acquire volume data by transmitting and receiving an ultrasound beam at a predetermined sampling cycle. The ultrasound diagnosis apparatus 300 may acquire the second ultrasound data after acquiring the first ultrasound data. For example, when the first ultrasound data is the volume data acquired at a first cycle, the second ultrasound data may be the volume data acquired at a cycle next to the first cycle. In general, a sampling cycle may have a unit of milliseconds (ms). Accordingly, movements of the object of interest and the probe may not be large between sampling cycles, and the first ultrasound data and the second ultrasound data may include images of the object of interest at relatively similar positions. Also, the image of the object of interest may include similar pixel values or voxel values.

Also, according to image processing capability of the ultrasound diagnosis apparatus 300, the second ultrasound data may be acquired after a predetermined cycle passes after the first ultrasound data is acquired. For example, when the image processing capability of the ultrasound diagnosis apparatus 300 is poor, the image process according to the present exemplary embodiment may not be performed on the volume data acquired between the first ultrasound data and the second ultrasound data. In other words, the ultrasound diagnosis apparatus 300 may not acquire the position of the object of interest in the volume data acquired between the first ultrasound data and the second ultrasound data. However, the volume data acquired between the first ultrasound data and the second ultrasound data may be displayed on the display 140 of FIG. 1. The ultrasound diagnosis apparatus 300 may improve efficiency by performing the image processing according to the present exemplary embodiment only on the first ultrasound data and the second ultrasound data. Also, since an interval between the time when the first ultrasound data is acquired and the time when the second ultrasound data is acquired is still short, the first ultrasound data and the second ultrasound data may include images of the object of interest at relatively similar positions. Also, the object of interest may have a similar pixel value or voxel value.

The controller 320 may detect a first position of the object of interest included in the object on the first ultrasound data. Also, the controller 320 may detect a second position of the object of interest based on the first position on the second ultrasound data. Also, the controller 320 may change a condition for transceiving an ultrasound beam based on the second position.

The controller 320 may perform at least one of the functions of the controller 170 and the image processor 120 of FIG. 1. The controller 320 may be at least one of the controller 170 and the image processor 120. Also, the controller 320 may be hardware separate from the controller 170 and the image processor 120.

The first position or the second position may be a predetermined position or area included the object of interest on the volume data. For example, the first position or the second position may be a center point, a right end point, a left end point, an upper end point or a lower end point of the object of interest. Also, the first position or the second position may be a predetermined area in the object of interest. Also, the first position or the second position may be presented by a coordinate value of a voxel on the volume data.

As described above, since the interval between the time when the first ultrasound data is acquired and the time when the second ultrasound data is acquired may be short, the first ultrasound data and the second ultrasound data may include images of the object of interest at relatively similar positions. For example, the controller 320 may detect the first position of the object of interest on the first ultrasound data. When the first position is a first coordinate value of a voxel on the volume data, the controller 320 may find the object of interest at around the first coordinate value on the second ultrasound data. Also, when finding the object of interest, the controller 320 may detect the second position.

The controller 320 may change the condition for transceiving an ultrasound beam based on the second position. The condition for transceiving an ultrasound beam may include at least one of a receiving depth of an ultrasound beam, a width of an ultrasound beam, a steering angle of an ultrasound beam, and a focusing position of an ultrasound beam.

For example, the controller 320 may adjust the receiving depth of an ultrasound beam to scan the second position. Also, the controller 320 may adjust the width of an ultrasound beam to scan the second position.

Also, the controller 320 may control an ultrasound beam to be output toward the second position. The controller 320 may change the steering angle of an ultrasound beam. The steering angle is an angle between the ultrasound beam and a surface made by transducers included in a probe. When the object of interest is inclined toward the right side on an ultrasound image, the controller 320 changes the steering angle so that the object of interest may be located at the center of the ultrasound image. Also, the controller 320 may change the focusing position of an ultrasound beam. Accordingly, the ultrasound diagnosis apparatus 300 may acquire a clear ultrasound image with respect to the object of interest.

Also, the controller 320 may change, in real time, the condition for transceiving an ultrasound beam. Accordingly, a user may check, in real time, an ultrasound image transceived according to a changed transceiving condition.

In the following description, an ultrasound diagnosis apparatus and a method of operating an ultrasound diagnosis apparatus according to an exemplary embodiment are described below in detail with reference to FIG. 3.

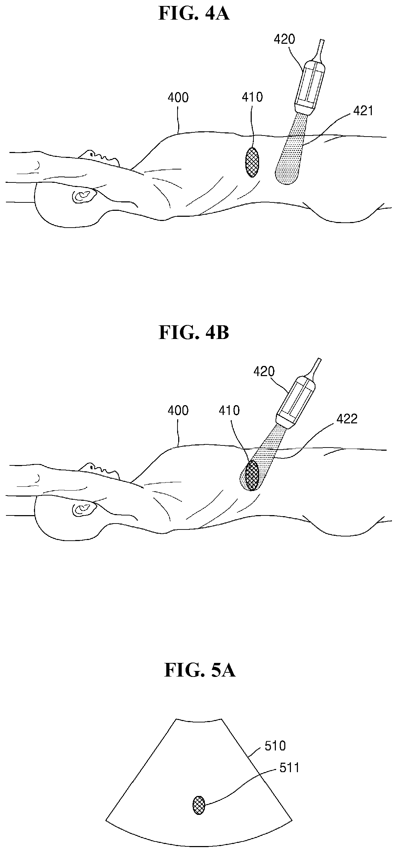

FIGS. 4A and 4B illustrate a process of acquiring ultrasound data according to an exemplary embodiment.

Referring to FIG. 4A, the ultrasound diagnosis apparatus 300 may acquire volume data by scanning an object 400 using a probe 420. The probe 420 may output an ultrasound beam 421. An output ultrasound beam 422 may be reflected by the object 400. The ultrasound diagnosis apparatus 300 receives a reflected signal, thereby acquiring the volume data.

Referring to FIG. 4A, since the ultrasound beam 421 does not point at an object of interest 410, the acquired volume data may not contain information related to the object of interest 410. The ultrasound diagnosis apparatus 300 may generate an ultrasound image based on the volume data. While checking the ultrasound image, the user may correct the position and angle of the probe.

Referring to FIG. 4B, the ultrasound diagnosis apparatus 300 may acquire volume data by scanning the object 400 using the probe 420. Since the ultrasound beam 422 points at the object of interest 410, the acquired volume data may contain information related to the object of interest 410. The ultrasound diagnosis apparatus 300 may generate an ultrasound image based on the volume data. When the probe 420 faces the object of interest 410, as illustrated in FIG. 4B, the ultrasound diagnosis apparatus 300 may acquire the volume data. For example, the volume data may be the first ultrasound data.

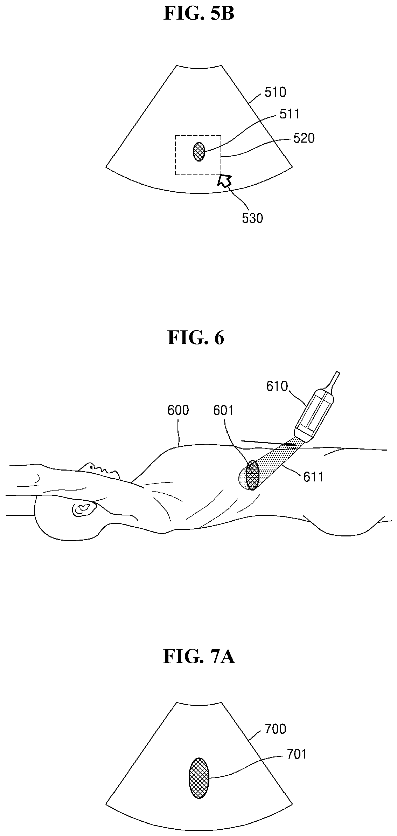

FIGS. 5A and 5B schematically illustrate ultrasound images acquired based on ultrasound data according to an exemplary embodiment.

When the probe 420 points the object of interest 410, as illustrated in FIG. 4B, an ultrasound image 510 may be acquired as illustrated in FIG. 5A. An image 511 of the object of interest may be displayed on the ultrasound image 510.

Referring to FIG. 5B, the ultrasound diagnosis apparatus 300 may acquire volume data, and the volume data may be first ultrasound data. The ultrasound diagnosis apparatus 300 may acquire the ultrasound image 510 based on the first ultrasound data. The ultrasound diagnosis apparatus 300 may include an input unit (not shown) that may receive an input from the user. The input unit may receive from the user an input to set a region of interest (ROI) on the first ultrasound image based on the first ultrasound data. The input unit may identically correspond to the input device 160 of FIG. 1.

The input unit may receive a user's input. The ultrasound diagnosis apparatus 300 may move a marker 530 based on the user's input on the ultrasound image 510. Also, the ultrasound diagnosis apparatus 300 may set a predetermined ROI 520. The ROI 520 may be an area including the image 511 of the object of interest.

The controller 320 may detect a first position of the image 511 of the object of interest in the ROI 520. For example, the ultrasound diagnosis apparatus 300 may acquire the image 511 of the object of interest by comparing the ROI 520 with a predetermined image. The predetermined image may be an image of the object of interest of an examinee that is previously acquired by the ultrasound diagnosis apparatus 300. Also, the predetermined image may be a reference image of the object of interest stored by the ultrasound diagnosis apparatus 300 where the object of interest is well displayed. The ultrasound diagnosis apparatus 300 may perform image processing on the ROI 520 in the ultrasound image 510 so that the image 511 of the object of interest is well displayed. For example, the ultrasound diagnosis apparatus 300 may acquire an outline by performing image processing on the ROI 520. Also, the ultrasound diagnosis apparatus 300 may acquire the ROI 520 as the image 511 of the object of interest.

The ultrasound diagnosis apparatus 300 may detect the first position of the object of interest based on the image 511 of the object of interest that is acquired. Since the first position is described above, a detailed description thereof is omitted.

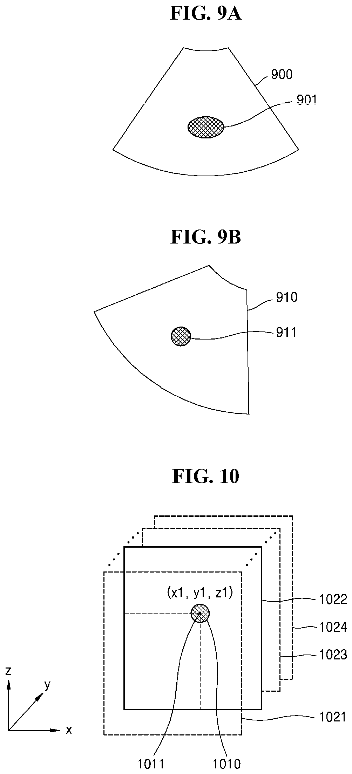

The ultrasound diagnosis apparatus 300 may detect a second position of the object of interest based on the first position. Also, the ultrasound diagnosis apparatus 300 may change the condition for transceiving an ultrasound beam based on the second position. Also, the ultrasound diagnosis apparatus 300 may change the condition for transceiving an ultrasound beam further based on the first position. In the following description, the operation of the ultrasound diagnosis apparatus 300 is described in detail with reference to FIGS. 10 to 16.

FIG. 10 illustrates first ultrasound data according to an exemplary embodiment.

The ultrasound diagnosis apparatus 300 may acquire a first coordinate value indicating the first position on the first ultrasound data. Also, the ultrasound diagnosis apparatus 300 may acquire a second coordinate value indicating the second position on the second ultrasound data. Also, the ultrasound diagnosis apparatus 300 may change the condition for transceiving an ultrasound beam based on a difference value between the first coordinate value and the second coordinate value.

Referring to FIG. 10, the first ultrasound data may include a plurality of pieces of two-dimensional data 1021, 1022, 1023, and 1024. Also, the ultrasound diagnosis apparatus 300 may acquire the ultrasound image 510 of FIGS. 5A and 5B based on the two-dimensional data 1022. In other words, the ultrasound image 510 of FIGS. 5A and 5B may correspond to the two-dimensional data 1022.

The pieces of two-dimensional data may signify parallel planes included in the volume data. Also, the two-dimensional data may be data of one slice included in the volume data.

The controller 320 may detect the first position of an object of interest 1010 in the ROI. Also, the controller 320 may acquire a first coordinate value indicating the first position on the first ultrasound data.

The first position may be a predetermined position or area included in the object of interest on the first ultrasound data. For example, the first position may be a center point, a right end point, a left end point, an upper end point, or a lower end point in the object of interest. Also, the first position may be a predetermined area in the object of interest. For example, the ultrasound diagnosis apparatus 300 may present the first position as a figure such as a circle or a rectangle in the object of interest. Also, the ultrasound diagnosis apparatus 300 may acquire certain point in the figure as the first position. Also, the first position may be indicated by a coordinate value of a voxel on the first ultrasound data.

As described above, the first position may be indicated by a coordinate value of a voxel in the first ultrasound data. Each of the two-dimensional data 1021, 1022, 1023, and 1024 may have different y coordinate values. For example, a y coordinate value of the two-dimensional data 1021 may be y0. Also, a y coordinate value of the two-dimensional data 1022 including the object of interest 1010 may be y1.

Each voxel in the two-dimensional data 1022 may have a coordinate value with respect to x and z axes. The first position of the object of interest 1010 may be a center point 1011 of the object of interest 1010. The center point 1011 may be calculated with an average of the coordinate values of all the voxels included in the object of interest 1010. The center point 1011 of the object of interest 1010 may have, for example, a coordinate value "(x1, z1)" in the two-dimensional data 1022. The ultrasound diagnosis apparatus 300 may acquire a coordinate value "(x1, y1, z1)" as the first position of the object of interest 1010.

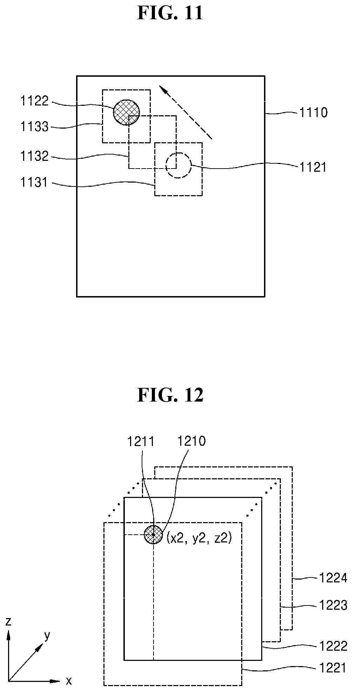

FIG. 11 illustrates a process in which the ultrasound diagnosis apparatus 300 according to an exemplary embodiment searches for a position of an object of interest in the second ultrasound data.

The ultrasound diagnosis apparatus 300 may acquire the second ultrasound data after a predetermined time passes after the first ultrasound data is acquired. For the predetermined time, the user may change the position of the probe. Also, while the probe may stand still, the object may move. The ultrasound diagnosis apparatus 300 may acquire the second ultrasound data after the position of the probe is changed or the object moves.

Since the predetermined time that is the interval between the time when the first ultrasound data is acquired and the time when the second ultrasound data is acquired is short, the first ultrasound data and the second ultrasound data may include images of the object of interest at relatively similar positions. The ultrasound diagnosis apparatus 300 may detect the position of the object of interest in the second ultrasound data based on the two-dimensional data 1110 included in the second ultrasound data. Also, the ultrasound diagnosis apparatus 300 may acquire the ultrasound image based on the second ultrasound data. While checking the ultrasound image, the user may check a process in which the ultrasound diagnosis apparatus 300 detects the object of interest. Also, the ultrasound diagnosis apparatus 300 may receive a user's input and detect the position of the object of interest in the second ultrasound data based on the user's input.

The ultrasound diagnosis apparatus 300 may detect the second position 1122 of the object of interest based on a first position 1121 of the object of interest. For example, since the predetermined time that is the interval between the time when the first ultrasound data is acquired and the time when the second ultrasound data is acquired is short, as described above, the second position 1122 may be detected around the first position 1121.

In detail, referring to FIGS. 10 and 11, the first position 1121 may be indicated by the coordinate value "(x1, y1, z1)" as described with reference to FIG. 10. The ultrasound diagnosis apparatus 300 may search for the object of interest around the coordinate value "(x1, y1, z1)" that is the first position 1121 on the second ultrasound data. For example, the ultrasound diagnosis apparatus 300 may detect whether the object of interest exists within a predetermined distance around the coordinate value "(x1, y1, z1)" on the second ultrasound data.

The ultrasound diagnosis apparatus 300 may search whether the object of interest exists in an area 1131 including the first position 1121 on the second ultrasound data. The ultrasound diagnosis apparatus 300 may detect a second position of the object of interest on the second ultrasound data based on a degree of correlation between at least one of pixel values and voxel values of the object of interest on the first ultrasound data and at least one of pixel values and voxel values on the second ultrasound data. To detect the second position, the ultrasound diagnosis apparatus 300 may determine whether the object of interest exists on the second ultrasound data.

Since a predetermined time between the time when the first ultrasound data is acquired and the time when the second ultrasound data is acquired is short, as described above, the first ultrasound data and the second ultrasound data may be similar to each other. Also, the pixel values included in an image of the object of interest on the first ultrasound data may be similar to the pixel values included in an image of the object of interest on the second ultrasound data. Accordingly, the ultrasound diagnosis apparatus 300 may detect, on the second ultrasound data, pixel values similar to the pixel values included in the image of the object of interest on the first ultrasound data.

For example, the ultrasound diagnosis apparatus 300 may determine whether the object of interest exists in the area 1131 by comparing an image of the area 1131 of the second ultrasound data and an image of the object of interest of the first ultrasound data. The area 1131 may be an area including a coordinate on the second ultrasound data corresponding to the coordinate of the position of the object of interest on the first ultrasound data.

When a degree of correlation between the pixel values included in the image of the area 1131 and the pixel values included in the image of the object of interest on the first ultrasound data is equal to or greater than a threshold value, the ultrasound diagnosis apparatus 300 may determine whether the object of interest exists in the area 1131. Whether the object of interest exists in the area 1131 may be determined by comparing the area 1131 and the ROI of FIG. 5B. The ultrasound diagnosis apparatus 300 may calculate a degree of correlation by using a statistical method such as correlation. However, the present exemplary embodiment is not limited thereto and various correlation degree measurement methods may be used therefor.

The ultrasound diagnosis apparatus 300 may acquire an outline of the image of the object interest in the first ultrasound data. Also, the ultrasound diagnosis apparatus 300 may compare the outline of the image shown in the area 1131 with the outline of the object of interest of the first ultrasound data. When the ultrasound diagnosis apparatus 300 compares only the outline, efficiency of data processing may be improved.

Also, the ultrasound diagnosis apparatus 300 may determine whether the object of interest exists in the area 1131 by comparing the area 1131 with a reference image of the object of interest where the object of interest is well displayed. Also, the ultrasound diagnosis apparatus 300 may determine whether the object of interest exists in the area 1131 considering the pieces of volume data acquired prior to the first ultrasound data.

As illustrated in FIG. 11, the ultrasound diagnosis apparatus 300 may determine that the object of interest does not exist in the area 1131. The ultrasound diagnosis apparatus 300 may determine whether the object of interest exists in other area around the area 1131. The ultrasound diagnosis apparatus 300 may determine whether the object of interest exists in a certain area around the area 1131. Also, the ultrasound diagnosis apparatus 300 may determine whether the object of interest exists in a certain area around the area 1131 by detecting the movement of the probe or based on the statistic data about the movement of the object.

Referring to FIG. 11, the ultrasound diagnosis apparatus 300 may determine whether the object of interest exists in an area 1132. The ultrasound diagnosis apparatus 300 may determine that the object of interest does not exist in the area 1132. However, the ultrasound diagnosis apparatus 300 may estimate that a part of the object of interest exists in the upper left corner of the area 1132. Accordingly, the ultrasound diagnosis apparatus 300 may determine whether the object of interest exists in an area 1133 at the upper left corner of the area 1132. Also, the ultrasound diagnosis apparatus 300 may determine that the object of interest exists in the area 1133. Also, the ultrasound diagnosis apparatus 300 may detect the second position of the object of interest existing in the area 1133.

Although FIG. 11 is described in two dimensions for convenience of explanation, the present exemplary embodiment is not limited thereto. The ultrasound diagnosis apparatus 300 may detect the second position of the object of interest on the second ultrasound data in three dimensions. The controller 320 may detect the second position of the object of interest on the second ultrasound data based on a degree of correlation between the voxel values of the object of interest on the first ultrasound data and the voxel values of the object of interest on the second ultrasound data. Also, the process of searching for the position of the object of interest on the second ultrasound data may be performed not only by the above-described method but also by various well-known methods.

FIG. 12 illustrates the second ultrasound data according to an exemplary embodiment.

Referring to FIG. 12, the controller 320 may detect the second position of an object of interest 1210 in an ROI. Also, the controller 320 may acquire a second coordinate value indicating the second position on the second ultrasound data.

The second position may be a predetermine position or area included in the object of interest on the second ultrasound data. For example, the second position may be a center point, a right end point, a left end point, an upper end point, or a lower end point in the object of interest. Also, the first position may be a predetermined area in the object of interest. Also, the second position may be a predetermined area in the object of interest. Also, the second position may be indicated by a coordinate value of a voxel on the second ultrasound data.

As described above, the second position may be indicated by a coordinate value of a voxel in the second ultrasound data. Each of a plurality of pieces of two-dimensional data 1221, 1222, 1223, and 1224 may have different y coordinate values. For example, a y coordinate value of the two-dimensional data 1221 may be y0. Also, a y coordinate value of the two-dimensional data 1222 including the object of interest 1210 may be y2.

Each voxel in the two-dimensional data 1222 may have a coordinate value with respect to x and z axes. The second position of the object of interest 1210 may be a center point 1211 of the object of interest. The center point 1211 of the object of interest may have, for example, a coordinate value "(x2, z2)" in the two-dimensional data 1222. The ultrasound diagnosis apparatus 300 may acquire a coordinate value "(x2, y2, z2)" as the first position of the object of interest 1210.

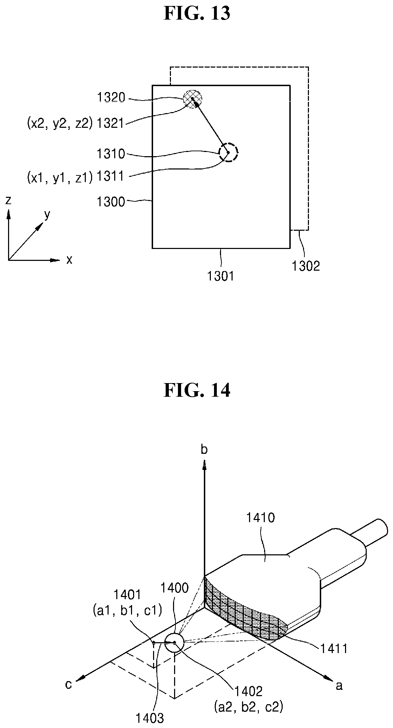

FIG. 13 illustrates a first position and a second position on volume data according to an exemplary embodiment.

Referring to FIG. 13, the ultrasound diagnosis apparatus 300 may include image information of an object of interest 1310 in one piece of two-dimensional data 1301 included in the first ultrasound data. Also, the ultrasound diagnosis apparatus 300 may acquire a voxel coordinate of a center point of the object of interest 1310 on the two-dimensional data 1301. A voxel coordinate of the center point of the object of interest 1310 of the first ultrasound data may be "(x1, y1,z1)".

Also, the ultrasound diagnosis apparatus 300 may include image information of an object of interest 1320 in one piece of two-dimensional data 1302 included in the second ultrasound data. Also, the ultrasound diagnosis apparatus 300 may acquire a voxel coordinate of a center point of the object of interest 1320 on the two-dimensional data 1302. A voxel coordinate of the center point of the object of interest 1320 of the second ultrasound data may be "(x2, y2, z2)".

The ultrasound diagnosis apparatus 300 may change the condition for transceiving an ultrasound beam based on a difference value between a voxel coordinate value indicating a first position on the first ultrasound data and a voxel coordinate value indicating a second position on the second ultrasound data. The difference value may be a difference or displacement of a coordinate value. Also, the difference value in the voxel coordinate value may be indicated by a vector that is "(x2-x1, y1-y2, z1-z3)". The ultrasound diagnosis apparatus 300 may change the condition for transceiving an ultrasound beam based on the direction and size of a vector.

FIG. 14 illustrates the first position and the second position in space according to an exemplary embodiment.

Referring to FIG. 14, the ultrasound diagnosis apparatus 300 may include a probe 1410. The probe 1410 may have a transducer array 1411. The ultrasound diagnosis apparatus 300 may steer an ultrasound beam by using the transducer array 1411. The ultrasound diagnosis apparatus 300 may have a coordinate of a voxel on the volume data correspond to a coordinate in space.

In FIGS. 10 to 13, the coordinate of a voxel on the volume data is indicated on an x-axis, a y-axis, and a z-axis. In FIG. 14, a coordinate in space may be indicated by an a-axis, a b-axis, and a c-axis. The origin of the a-axis, the b-axis, and the c-axis may be a lower left point of the transducer array 1411. However, the present exemplary embodiment is not limited thereto, and the original of the a-axis, the b-axis, and the c-axis may be the center, lower left, or upper right point of the transducer array 1411. The unit of the a-axis, the b-axis, and the c-axis may be, for example, mm or cm, which is a unit of length. The x-axis may correspond to the a-axis. Also, the y-axis may correspond to the b-axis. Also, the z-axis may correspond to the c-axis.

The ultrasound diagnosis apparatus 300 may have the first position and the second position that are coordinates of voxels on the volume data of FIG. 13 correspond to coordinates in space. For example, the ultrasound diagnosis apparatus 300 may have mapping data that transforms a coordinate of a voxel to a space coordinate and a transformation function that transforms a coordinate of a voxel to a space coordinate. The ultrasound diagnosis apparatus 300 may have the first position on the first ultrasound data correspond to a position 1401 in space. Also, a coordinate of the position 1401 in space may be "(a1, b1, c1)". Also, the ultrasound diagnosis apparatus 300 may have the second position on the second ultrasound data correspond to a position 1402 in space. Also, a coordinate of the position 1402 in space may be "(a2, b2, c2)".

The ultrasound diagnosis apparatus 300 may change the condition for transceiving an ultrasound beam based on a difference value between a space coordinate value corresponding to the first position on the first ultrasound data and a space coordinate value corresponding to the second position on the second ultrasound data. The difference value between the space coordinate values may be indicated by a vector that may be a coordinate "(a2-a1, b2-b1, c2-c1)". The ultrasound diagnosis apparatus 300 may change the condition for transceiving an ultrasound beam based on the difference value between the space coordinate values. Also, the ultrasound diagnosis apparatus 300 may focus an ultrasound beam at an object of interest 1400 based on the difference value between the space coordinate values.

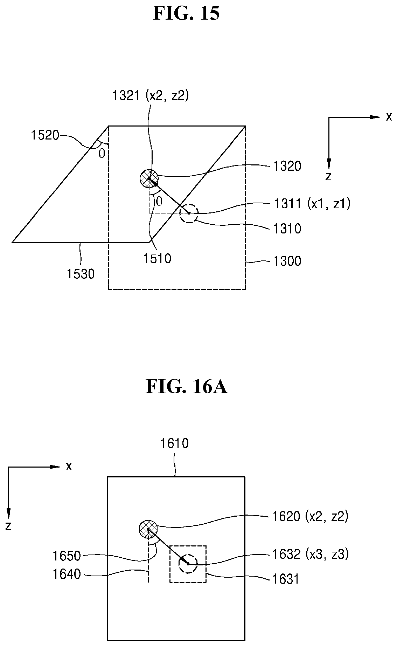

FIG. 15 is a view for explaining a method of changing a transceiving condition of an ultrasound diagnosis apparatus according to an exemplary embodiment.

The controller 320 may change the condition for transceiving an ultrasound beam based on the second position. Also, the controller 320 may change the condition for transceiving an ultrasound beam further based on the first position. Also, the controller 320 may acquire a first coordinate value indicating the first position on the first ultrasound data. Also, the controller 320 may acquire a second coordinate value indicating the second position on the second ultrasound data. Also, the controller 320 may change the condition for transceiving an ultrasound beam based on a difference value between the first coordinate value and the second coordinate value.

Referring to FIGS. 13 and 15, the position of the object of interest on the pieces of volume data may be changed. The ultrasound diagnosis apparatus 300 may detect a first position 1311 of the object of interest 1310 on the first ultrasound data. Also, the ultrasound diagnosis apparatus 300 may detect a second position 1321 of the object of interest 1320 on the second ultrasound data. The ultrasound diagnosis apparatus 300 may change the condition for transceiving an ultrasound beam based on the difference value between the first position and the second position.

The volume data may include a plurality of pieced of two-dimensional data. In FIG. 15, for convenience of explanation, a case in which the first position and the second position exist on the same two-dimensional data 1300 is illustrated. The ultrasound diagnosis apparatus 300 may acquire the first position 1311 of the object of interest 1310 on the two-dimensional data 1300. The first position 1311 may be a center point of the object of interest 1310. Also, the first position 1311 may be indicated by a coordinate value "(x1, z1)". Also, the ultrasound diagnosis apparatus 300 may acquire the second position 1321 of the object of interest 1320 on the two-dimensional data 1300. The second position 1321 may be a center point of the object of interest 1320. Also, the second position 1321 may be indicated by a coordinate value "(x2, z2)".

The ultrasound diagnosis apparatus 300 may acquire a difference value between the first position 1311 and the second position 1321. The difference value may be a vector that may be indicated by a coordinate "(x2-x1, z2-z1)". The ultrasound diagnosis apparatus 300 may acquire an angle 1510 formed between the z axis and the vector. The ultrasound diagnosis apparatus 300 may calculate the angle 1510 by using a function "atan(|x2-x1 |/|z2-z1|)". The ultrasound diagnosis apparatus 300 may change a steering angle 1520 of an ultrasound beam based on the angle 1510. Also, the ultrasound diagnosis apparatus 300 may acquire the volume data by using the changed steering angle 1520. Also, the acquired volume data may include two-dimensional data 1530.

FIGS. 16A and 16B are views for explaining a method of changing a transceiving condition of an ultrasound diagnosis apparatus according to an exemplary embodiment.

The controller 320 may change the condition for transceiving an ultrasound beam based on the second position. Also, the controller 320 may acquire a second coordinate value indicating the second position on the second ultrasound data. Also, the controller 320 may change the condition for transceiving an ultrasound beam based on the difference value between the second position and a preset position.

Referring to FIG. 16A, the position of the object of interest may be changed on the pieces of volume data. The ultrasound diagnosis apparatus 300 may detect a second position 1620 of the object of interest on the second ultrasound data. As described with reference to FIGS. 10 and 11, the second position may be detected based on the first position.

The ultrasound diagnosis apparatus 300 may have a preset position. For example, the ultrasound diagnosis apparatus 300 may store the preset position in the memory 150 of FIG. 1. Also, the ultrasound diagnosis apparatus 300 may acquire the preset position based on a user's input. The preset position may be a position on the volume data where the object of interest is observed well. Also, the preset position may be a position on the ultrasound image where the object of interest is observed well. The preset position may be an area 1631 or a position 1632.

When the preset position is the area 1631, the ultrasound diagnosis apparatus 300 may change the condition for transceiving an ultrasound beam so that at least a part of the object of interest enters the area 1631. Also, when the preset position is a position 1632, the ultrasound diagnosis apparatus 300 may change the condition for transceiving an ultrasound beam so that the object of interest is located at the position 1632.

The ultrasound diagnosis apparatus 300 may acquire a coordinate value indicating the second position to be "(x2, z2)". Also, the ultrasound diagnosis apparatus 300 may acquire a coordinate value indicating the preset position to be "(x3, z3)". The ultrasound diagnosis apparatus 300 may acquire a difference value between a coordinate value indicating the second position and a coordinate value indicating the preset position. For example, a vector indicating the difference value may be "(x3-x2, z3-z2)".

The ultrasound diagnosis apparatus 300 may acquire an angle 1650 formed between the z-axis and the vector. The ultrasound diagnosis apparatus 300 may calculate the angle 1650 by using a function "atan(|x3-x2|/|z3-z2|)".

Referring to FIG. 16B, the ultrasound diagnosis apparatus 300 may change a steering angle 1660 of an ultrasound beam based on the angle 1650. Also, the ultrasound diagnosis apparatus 300 may acquire the volume data by using the changed steering angle 1660. Also, the acquired volume data may include two-dimensional data 1670.

FIG. 6 illustrates an operation of an ultrasound diagnosis apparatus according to an exemplary embodiment.

Referring to FIG. 6, the user may move the position of a probe 610. The ultrasound diagnosis apparatus 300 may change the condition for transceiving an ultrasound beam 611 as described above with reference to FIGS. 10 to 16. Accordingly, although the probe 610 is moved, the ultrasound beam 611 may point at an object of interest 601.

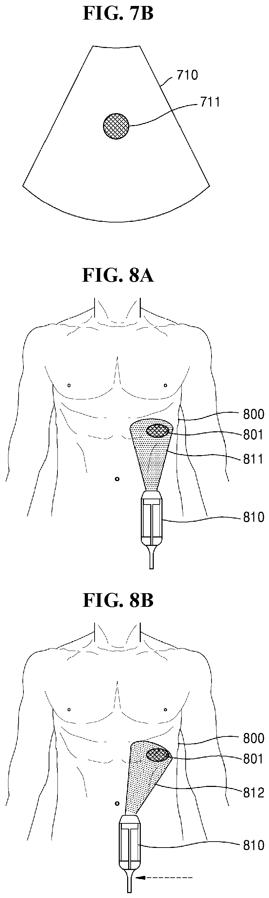

FIGS. 7A and 7B schematically illustrate ultrasound images 700 and 710 according to an exemplary embodiment.

As described with reference to FIG. 6, the ultrasound diagnosis apparatus 300 may change the condition for transceiving an ultrasound beam. FIG. 7A illustrates the ultrasound image 700 acquired by the ultrasound diagnosis apparatus 300 at a position of the probe 610 in FIG. 6. The ultrasound image 510 of FIG. 5A may be the first ultrasound image based on the first data, and the ultrasound image 700 of FIG. 7A may be the second ultrasound image based on the second data.

An image 701 of the object of interest may be displayed on the ultrasound image 700. The image 701 of the object of interest and the image 511 of the object of interest of FIG. 5A may be images of the object of interest viewed at different angles. The image 701 of the object of interest and the image 511 of the object of interest of FIG. 5A are different from each other because the position of the probe is moved and the ultrasound diagnosis apparatus 300 changes the condition for transceiving an ultrasound beam. The mage 701 of the object of interest is an image viewed from the position of the probe 610 of FIG. 6. Also, the image 511 of the object of interest of FIG. 5A is an image viewed from the position of the probe 420 of FIG. 4B. The user may easily acquire an image of the object of interest viewed from a different position only by changing the position of the probe.

FIG. 7B illustrates the ultrasound image 710 acquired by the ultrasound diagnosis apparatus 300 at a position of the probe 610 in FIG. 6. The ultrasound image 510 of FIG. 5A may be the first ultrasound image based on the first data, and the ultrasound image 710 of FIG. 7B may be the second ultrasound image based on the second data.

An image 711 of the object of interest may be displayed on the ultrasound image 710. The image 711 of the object of interest and the image 511 of the object of interest of FIG. 5A may be images of the object of interest viewed at different angles.

The display 140 of FIG. 1 may display the second ultrasound image including the object of interest based on the second ultrasound data. Also, the display 140 may display the second ultrasound image by changing at least one of the shape, size, and position of the first ultrasound image according to the change of the condition for transceiving an ultrasound beam.

The ultrasound diagnosis apparatus 300 may change the condition for transceiving an ultrasound beam that includes at least one of the receiving depth of an ultrasound beam, the width of an ultrasound beam, the steering angle of an ultrasound beam, and the focusing position of an ultrasound beam.

The ultrasound diagnosis apparatus 300 may change the condition for transmitting an ultrasound beam so that the ultrasound beam may reach deep in the object. For example, the ultrasound diagnosis apparatus 300 may transmit an ultrasound beam having a low frequency. Also, after transmitting an ultrasound beam, the ultrasound diagnosis apparatus 300 may receive an ultrasound echo signal reflected from the object. The ultrasound diagnosis apparatus 300 may receive only an ultrasound echo signal reflected at a distance less than a predetermined distance. The predetermined distance may be a receiving depth of an ultrasound beam. Also, the ultrasound diagnosis apparatus 300 may acquire the ultrasound image 710 based on the ultrasound echo signal reflected at a distance less than a receiving depth of an ultrasound beam. The receiving depth of an ultrasound beam may be related to a vertical length of the ultrasound image 710.

The width of an ultrasound beam may be related to the horizontal length of the ultrasound image 710. The ultrasound diagnosis apparatus 300 may determine the horizontal width of an ultrasound beam by using transducers. Also, the ultrasound diagnosis apparatus 300 may receive an ultrasound echo signal having a width less than a predetermined width among the reflected ultrasound echo signal. Also, the ultrasound diagnosis apparatus 300 may acquire the ultrasound image 710 based on an ultrasound echo signal having a width less than the predetermined width.

Also, the steering angle of an ultrasound beam may be related to the inclination of the ultrasound image 710. The ultrasound diagnosis apparatus 300 may transmit/receive an ultrasound beam at a predetermined steering angle. Since the steering angle of an ultrasound beam in the ultrasound diagnosis apparatus 300 is described above with reference to FIG. 3, a detailed description thereof is omitted.

Also, the focusing position of an ultrasound beam may be related to an area having a high resolution on the ultrasound image 710. In general, an area around a position at which the ultrasound beam is focused is an area having a high resolution on the ultrasound image 710.

Also, the ultrasound image 710 and the ultrasound image 510 of FIG. 5A may have different image widths and different vertical lengths. For example, the vertical length of the ultrasound image 710 may be longer than that of the ultrasound image 510 of FIG. 5A. This is because an arrival distance of the ultrasound beam 611 of FIG. 6 is longer than that of the ultrasound beam 422 of FIG. 4B.

FIGS. 8A and 8B illustrate a process of acquiring ultrasound data according to an exemplary embodiment.

FIG. 4B illustrates the object 400 viewed from a lateral side thereof, and FIG. 8A illustrates an object 800 viewed from a front side thereof. The position of a probe 810 of FIG. 8A corresponds to that of the probe 420 of FIG. 4B. An ultrasound beam 811 of FIG. 8A corresponds to that of the ultrasound beam 422 of FIG. 4B.

Referring to FIG. 8A, the ultrasound diagnosis apparatus 300 may acquire volume data by scanning the object 800 by using the probe 810. Since the ultrasound beam 811 points at the object of interest 801, the acquired volume data may contain information related to the object of interest 801. The ultrasound diagnosis apparatus 300 may generate an ultrasound image based on the volume data.

Referring to FIG. 8B, the user may move the position of the probe 810. The ultrasound diagnosis apparatus 300 may change the condition for transceiving an ultrasound beam 812 as described with reference to FIGS. 10 to 16. Accordingly, even when the probe 810 is moved, the ultrasound beam 812 may continuously points at the object of interest 801.

FIGS. 9A and 9B schematically illustrate ultrasound images 900 and 910 acquired based on ultrasound data according to an exemplary embodiment.

As described in FIG. 8B, the ultrasound diagnosis apparatus 300 may change the condition for transceiving an ultrasound beam. FIG. 9A illustrates the ultrasound image 900 acquired by the ultrasound diagnosis apparatus 300 at a position of the probe 810 of FIG. 8B. The ultrasound image 510 of FIG. 5A may be the first ultrasound image based on the first data, and the ultrasound image 900 of FIG. 9A may be the second ultrasound image based on the second data. An image 901 of the object of interest may be displayed on the ultrasound image 900. The image 901 of the object of interest and the image 511 of the object of interest of FIG. 5A may be images of the object of interest viewed at different angles. The user may easily acquire images of the object of interest viewed from different positions by only changing the position of the probe. The descriptions related to FIG. 9A, which are already presented above with reference to FIG. 7A, are omitted.

FIG. 9B illustrates the ultrasound image 910 acquired by the ultrasound diagnosis apparatus 300 at a position of the probe 810 of FIG. 8B. The ultrasound image 510 of FIG. 5A may be the first ultrasound image based on the first data, and the ultrasound image 910 of FIG. 9B may be the second ultrasound image based on the second data. An image 911 of the object of interest may be displayed on the ultrasound image 910. The image 911 of the object of interest and the image 511 of the object of interest of FIG. 5A may be images of the object of interest viewed at different angles.

Also, the display 140 of FIG. 1 may display the second ultrasound image including the object of interest based on the second ultrasound data. Also, the display 140 may display the second ultrasound image by changing at least one of the shape, size, and position of the first ultrasound image according to the change of the condition for transceiving an ultrasound beam.

For example, the ultrasound image 910 and the ultrasound image 510 of FIG. 5A may have different inclinations. The ultrasound image 910 may be inclined to the right compared to the ultrasound image 510 of FIG. 5A. As illustrated in FIG. 8, the ultrasound diagnosis apparatus 300 may change the steering angle of the ultrasound beam 812. The ultrasound diagnosis apparatus 300 may display the ultrasound image 510 to be inclined to the right based on the changed steering angle. However, the present exemplary embodiment is not limited thereto, and the ultrasound diagnosis apparatus 300 may display the ultrasound image 510 not to be inclined through image processing. Also, although a case of changing the steering angle is described in the above description, as the ultrasound diagnosis apparatus 300 changes the condition for transceiving an ultrasound beam, such as the receiving depth of an ultrasound beam, the width of an ultrasound beam, the steering angle of an ultrasound beam, and the focusing position of an ultrasound beam, the display 140 may display the second ultrasound image by changing at least one of the shape, size, and position of the second ultrasound image.

FIG. 17 is a flowchart for describing a method of operating an ultrasound diagnosis apparatus according to an exemplary embodiment.

An operation S1710 may be performed by the data acquirer 310. An operation S1720 may be performed by the controller 320. An operation S1730 may be performed by the data acquirer 310. An operation S1740 may be performed by the controller 320. An operation S1750 may be performed by the controller 320.

In the operation S1710, the ultrasound diagnosis apparatus 300 according to the present exemplary embodiment may acquire first ultrasound data about an object including an object of interest. Also, in the operation S1720, the ultrasound diagnosis apparatus 300 may detect a first position of the object of interest on the first ultrasound data. Also, in the operation S1730, the ultrasound diagnosis apparatus 300 may acquire second ultrasound data about the object. In the operation S1740, the ultrasound diagnosis apparatus 300 may detect a second position of the object of interest on the second ultrasound data. In the operation S1750, the ultrasound diagnosis apparatus 300 may change the condition for an ultrasound beam transmitted toward the object based on the second position.

Also, the detecting of the second position may be based on a degree of correlation of a pixel value or a voxel value of the object of interest on the first ultrasound data.

Also, in the changing of the transceiving condition, the condition for transceiving an ultrasound beam may be changed further based on the first position.

Also, the detecting of the first position may include acquiring a first coordinate value indicating the first position on the first ultrasound data. Also, the detecting of the second position may include acquiring a second coordinate value indicating the second position on the second ultrasound data. Also, in the changing of the transceiving condition, the condition for transceiving an ultrasound beam may be changed based on a difference value between the first coordinate value and the second coordinate value.

Also, the detecting of the first position may include acquiring a coordinate value of a center point of the object of interest on the first ultrasound data as the first coordinate value. Also, the detecting of the second position may include acquiring a coordinate value of a center point of the object of interest on the second ultrasound data as the second coordinate value.

Also, the changing of the transceiving condition may include changing the condition for transceiving an ultrasound beam based on a difference value between the second position and a preset position.

Also, the method of operating an ultrasound diagnosis apparatus according to the present exemplary embodiment may further include receiving a user's input for setting an ROI on the first ultrasound image based on the first ultrasound data. Also, the detecting of the first position may further include detecting the first position of the object of interest in the ROI.

Also, the condition for transceiving an ultrasound beam may include at least one of the receiving depth of an ultrasound beam, the width of an ultrasound beam, the steering angle of an ultrasound beam, and the focusing position of an ultrasound beam.

Also, the changing of the condition for an ultrasound beam transmitted toward the object may be performed in real time.

Also, the method of operating an ultrasound diagnosis apparatus according to the present exemplary embodiment may further include displaying a second ultrasound image including the object based on the second ultrasound data, and displaying the second ultrasound image by changing at least one of the shape, size, and position of the first ultrasound image according to the change of the condition for transceiving an ultrasound beam.

Also, a program for embodying the method of operating an ultrasound diagnosis apparatus according to the present exemplary embodiment may be recorded on a computer-readable recording medium.

As described above, the ultrasound diagnosis apparatus according to the above-described exemplary embodiment may easily acquire an ultrasound image of the object of interest at a different angle or the movement of a probe. Also, an ultrasound image may be easily acquired with respect to the object of interest by tracking the position of the object of interest.

Hardware may include at least one of a processor and memory. The term "processor" may be interpreted in a broader sense to include a central processing unit (CPU), a microprocessor, a digital signal processor (DSP), a controller, a microcontroller, state machine, etc. In some environments, the "processor" may refer to an application-specific integrated circuit (ASIC), a programmable logic device (PLD), a field-programmable gate array (FPGA), etc. The term "processor" may refer to, for example, a combination of processing devices such as a combination of a DSP and a microprocessor, a combination of a plurality of microprocessors, a combination of one or more microprocessors coupled to a DSP core, or a combination of other structures, etc.

The term "memory" may be interpreted in a broad sense to include any electronic component capable of storing electronic information. The term "memory" may refer to various types of processor-readable medium such as random access memory (RAM), read-only memory (ROM), non-volatile RAM (NVRAM), programmable ROM (PROM), erasable-PROM (EPROM), electrically erasable ROM (EEPROM), flash memory, a magnetic or optical data storage device, registers, etc. While the processor is capable of reading out information from memory and/or writing information to the memory, the memory is said to be in an electronic communication state with a processor. The memory integrated on the processor is in an electronic communication state with the processor.

The terms "commands" and "codes" may be interpreted in a broad sense to include any type of computer-readable text(s). For example, the terms "commands" and "codes" may refer to one or more programs, routines, sub-routines, procedures, etc. The terms "commands" and "codes" may include a single computer-readable text or many computer-readable texts

The present invention can be implemented as a method, an apparatus, and a system. When the present invention is implemented in software, its component elements are code segments that execute necessary operations. Programs or code segments can be stored in processor readable media and can be transmitted via a computer data signal that is combined with a carrier wave in a transmission medium or in a communication network. The processor readable medium can be any medium that can store or transmit data. Examples of the processor readable medium include electronic circuits, semiconductor memory devices, ROMs, flash memories, erasable ROMs (EROMs), floppy disks, optical disks, hard disks, optical fibers, radio frequency (RF) networks, etc. The computer data signal can be any signal that can be transmitted via transmission media, such as electronic network channels, optical fibers, air, an electronic field, RF networks, etc.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present inventive concept have been described with reference to the figures, it will be understood by those of ordinary skill in the art that various changes in form and details may be made therein without departing from the spirit and scope of the present inventive concept as defined by the following claims.

## Claims

1. An ultrasound diagnosis apparatus (300) comprising:
a data acquirer (310) configured for acquiring first ultrasound data and subsequent second ultrasound data with respect to an object (400);
a controller (320) configured for detecting a first position of an object of interest (410) included in the object (400) on the first ultrasound data, detecting a second position of the object of interest (410) based on the first position on the second ultrasound data, changing a condition for transceiving an ultrasound beam (421) based on the second position, and controlling to acquire an image of the object of interest based on the changed condition for transceiving an ultrasound beam (421); and
a display configured for displaying the acquired image of the object of interest based on the changed condition for transceiving by changing at least one of the shape, size and position of a first ultrasound image including the object of interest (410) based on the first ultrasound data according to the changed condition for transceiving an ultrasound beam, wherein the changed condition for transceiving an ultrasound beam (421) comprises a steering angle of an ultrasound beam (421), the image of the object of interest (410) based on the changed condition for transceiving an ultraousound beam (421) and the first ultrasound image being images of the object viewed at different angles.

2. The ultrasound diagnosis apparatus (300) of claim 1, wherein the controller (320) is configured to detect the second position of the object of interest (410) on the second ultrasound data based on a degree of correlation between at least one of pixel values and voxel values of the object of interest (410) on the first ultrasound data and at least one of pixel values and voxel values on the second ultrasound data.

3. The ultrasound diagnosis apparatus (300) of claim 1, wherein the controller (320) is configured to change the condition for transceiving an ultrasound beam (421) further based on the first position.

4. The ultrasound diagnosis apparatus (300) of claim 3, wherein the controller (320) is configured to acquire a first coordinate value indicating the first position on the first ultrasound data, acquire a second coordinate value indicating the second position on the second ultrasound data, and change the condition for transceiving an ultrasound beam (421) based on a difference value between the first coordinate value and the second coordinate value.

5. The ultrasound diagnosis apparatus (300) of claim 4, wherein the controller (320) is configured to acquire a coordinate value of a center point of the object of interest (410) on the first ultrasound data, as the first coordinate value, and a coordinate value of a center point of the object of interest (410) on the second ultrasound data, as the second coordinate value.

6. The ultrasound diagnosis apparatus (300) of claim 1, wherein the controller (320) is configured to change the condition for transceiving an ultrasound beam (421) based on a difference value between the second position and a preset position.

7. The ultrasound diagnosis apparatus (300) of claim 1, further comprising an input unit configured for receiving a user's input for setting a region of interest (ROI) on the first ultrasound image based on the first ultrasound data,
wherein the controller (320) is configured to detect the first position of the object of interest (410) in the ROI.

8. The ultrasound diagnosis apparatus (300) of claim 1, wherein the condition for transceiving an ultrasound beam (421) comprises at least one of a receiving depth of an ultrasound beam (421), a width of an ultrasound beam (421) and a focusing position of an ultrasound beam (421).

9. The ultrasound diagnosis apparatus (300) of claim 1, wherein the controller (320) is configured to change in real time the condition for transceiving an ultrasound beam (421) transmitted toward the object (400).

10. A method of operating an ultrasound diagnosis apparatus (300), the method comprising:
acquiring first ultrasound data with respect to an object (400) including an object of interest (410);
detecting a first position of the object of interest (410) on the first ultrasound data;
acquiring second ultrasound data with respect to the object (400);
detecting a second position of the object of interest (410) based on the first position on the second ultrasound data;
changing a condition for transceiving an ultrasound beam (421) transmitted toward the object (400) based on the second position;
acquiring an image of the object of interest (410) based on the changed condition for transceiving an ultrasound beam (421); and
displaying the acquired image of the object of interest based on the changed conditions for transceiving by changing at least one of the shape, size and position of a first ultrasound image including the object of interest based on the first ultrasound data according to the changed conditions for transceiving an ultrasound beam, wherein the changed condition for transceiving an ultrasound beam (421) comprises a steering angle of an ultrasound beam (421), the image of the object of interest (410) based on the changed condition for transceiving an ultrasound beam (421) and the first ultrasound image being images of the object viewed at different angles.

11. The method of claim 10, wherein the detecting of the second position is based on a degree of correlation between at least one of pixel values and voxel values of the object of interest (410) on the first ultrasound data and at least one of pixel values and voxel values on the second ultrasound data.

12. The method of claim 10, wherein, in the changing of the condition for transceiving an ultrasound beam (421), the condition for transceiving an ultrasound beam (421) is changed further based on the first position,
wherein the detecting of the first position comprises acquiring a first coordinate value indicating the first position on the first ultrasound data, the detecting of the second position comprises acquiring a second coordinate value indicating the second position on the second ultrasound data, and the changing of the condition for transceiving an ultrasound beam (421) transmitted toward the object (400) comprises changing the condition for transceiving an ultrasound beam (421) based on a difference value between the first coordinate value and the second coordinate value, and
wherein the detecting of the first position comprises acquiring a coordinate value of a center point of the object of interest (410) on the first ultrasound data, as the first coordinate value, and the detecting of the second position comprises a coordinate value of a center point of the object of interest (410) on the second ultrasound data, as the second coordinate value.

13. The method of claim 10, wherein the changing of the condition for transceiving an ultrasound beam (421) transmitted toward the object (400) comprises changing the condition for transceiving an ultrasound beam (421) based on a difference value between the second position and a preset position.

14. A non-transitory computer readable storage medium having stored thereon a program, which when executed on an ultrasound diagnostic apparatus, performs the method defined in any of claims 10-13.

## Patentansprüche

1. Ultraschalldiagnosevorrichtung (300), welche Folgendes aufweist:
Eine Datenerlangungseinrichtung (310), die dafür vorgesehen ist, erste Ultraschalldaten und nachfolgende zweite Ultraschalldaten in Bezug auf ein Objekt (400) zu erlangen;
ein Steuergerät (320), das dafür vorgesehen ist, eine erste Position eines interessierenden Objekts (410), das in dem Objekt (400) enthalten ist, auf den ersten Ultraschalldaten zu detektieren, eine zweite Position des interessierenden Objekts (410) basierend auf der ersten Position auf den zweiten Ultraschalldaten zu detektieren, eine Bedingung bzw. einen Zustand zum Senden und Empfangen eines Ultraschallstrahls (421) basierend auf der zweiten Position zu ändern und das Erlangen eines Bilds des interessierenden Objekts basierend auf der geänderten Bedingung zum Senden und Empfangen eines Ultraschallstrahls (421) zu steuern; und
eine Anzeige, die dafür vorgesehen ist, das erlangte Bild des interessierenden Objekts basierend auf der geänderten Bedingung zum Senden um Empfangen durch Ändern wenigstens eines der Form, Größe und Position eines ersten Ultraschallbilds, das das interessierende Objekt (410) enthält, basierend auf den ersten Ultraschalldaten gemäß der geänderten Bedingung zum Senden und Empfangen eines Ultraschallstrahls anzuzeigen,
wobei die geänderte Bedingung zum Senden und Empfangen eines Ultraschallstrahls (421) einen Steuerwinkel eines Ultraschallstrahls (421) enthält, und wobei das Bild des interessierenden Objekts (410), das auf der geänderten Bedingung zum Senden und Empfangen eines Ultraschallstrahls (421) basiert, und das erste Ultraschallbild als Bilder des Objekts, das in unterschiedlichen Winkeln betrachtet wird, ausgebildet ist.

2. Ultraschalldiagnosevorrichtung (300) nach Anspruch 1, wobei das Steuergerät (320) dafür vorgesehen ist, die zweite Position des interessierenden Objekts (410) auf den zweiten Ultraschalldaten basierend auf einem Grad der Korrelation zwischen wenigstens einem von Pixelwerten und Voxelwerten des interessierenden Objekts (410) auf den ersten Ultraschalldaten und wenigstens eines von Pixelwerten und Voxelwerten auf den zweiten Ultraschalldaten zu detektieren.

3. Ultraschalldiagnosevorrichtung (300) nach Anspruch 1, wobei das Steuergerät (320) dafür vorgesehen ist, die Bedingung zum Senden und Empfangen eines Ultraschallstrahls (421) des Weiteren basierend auf der ersten Position zu ändern.

4. Ultraschalldiagnosevorrichtung (300) nach Anspruch 3, wobei das Steuergerät (320) dafür vorgesehen ist, einen ersten Koordinatenwert, der die erste Position auf den ersten Ultraschalldaten anzeigt, zu erlangen, einen zweiten Koordinatenwert, der die zweite Position auf den zweiten Ultraschalldaten anzeigt, zu erlangen, und die Bedingung zum Senden und Empfangen eines Ultraschallstrahls (421) basierend auf einem Differenzwert zwischen dem ersten Koordinatenwert und dem zweiten Koordinatenwert zu ändern.

5. Ultraschalldiagnosevorrichtung (300) nach Anspruch 4, wobei das Steuergerät (320) dafür vorgesehen ist, einen Koordinatenwert eines Mittelpunkts des interessierenden Objekts (410) auf den ersten Ultraschalldaten als den ersten Koordinatenwert und einen Koordinatenwert eines Mittelpunkts des interessierenden Objekts (410) auf den zweiten Ultraschalldaten als den zweiten Koordinatenwert zu erlangen.

6. Ultraschalldiagnosevorrichtung (300) nach Anspruch 1, wobei das Steuergerät (320) dafür vorgesehen ist, die Bedingung zum Senden und Empfangen eines Ultraschallstrahls (421) basierend auf einem Differenzwert zwischen der zweiten Position und einer voreingestellten Position zu ändern.

7. Ultraschalldiagnosevorrichtung (300) nach Anspruch 1, welche des Weiteren eine Eingabeeinheit aufweist, die dafür vorgesehen ist, die Eingabe eines Benutzers zum Einstellen eines interessierenden Bereichs (ROI) auf dem ersten Ultraschallbild basierend auf den ersten Ultraschalldaten zu empfangen, wobei das Steuergerät (320) dafür vorgesehen ist, die erste Position auf dem interessierenden Objekt (410) in dem ROI zu detektieren.

8. Ultraschalldiagnosevorrichtung (300) nach Anspruch 1, wobei die Bedingung zum Senden und Empfangen eines Ultraschallstrahls (421) wenigstens eines von einem Empfangen der Tiefe eines Ultraschallstrahls (421), einer Breite eines Ultraschallstrahls (421) und einer Fokusposition eines Ultraschallstrahls (421) enthält.

9. Ultraschalldiagnosevorrichtung (300) nach Anspruch 1, wobei das Steuergerät (320) dafür vorgesehen ist, die Bedingung zum Senden und Empfangen eines Ultraschallstrahls (421), der in Richtung des Objekts (400) übertragen wird, in Echtzeit zu ändern.

10. Verfahren zum Betreiben einer Ultraschalldiagnosevorrichtung (300), wobei das Verfahren Folgendes aufweist:
Erlangen erster Ultraschalldaten in Bezug auf ein Objekt (400), das ein interessierendes Objekt (410) enthält;
Detektieren einer ersten Position des interessierenden Objekts (410) auf den ersten Ultraschalldaten;
Erlangen zweiter Ultraschalldaten in Bezug auf das Objekt (400);
Detektieren einer zweiten Position des interessierenden Objekts (410) basierend auf der ersten Position auf den zweiten Ultraschalldaten;
Ändern einer Bedingung bzw. eines Zustands zum Senden und Empfangen eines Ultraschallstrahls (421), der basierend auf der zweiten Position in Richtung des Objekts (400) übertragen wird;
Erlangen eines Bilds des interessierenden Objekts (410) basierend auf der geänderten Bedingung zum Senden und Empfangen eines Ultraschallstrahls (421); und
Anzeigen des erlangten Bilds des interessierenden Objekts basierend auf den geänderten Bedingungen zum Senden und Empfangen durch Ändern wenigstens eines der Form, Größe und Position eines ersten Ultraschallbilds, das das interessierende Objekt enthält, basierend auf den ersten Ultraschalldaten gemäß der geänderten Bedingungen zum Senden und Empfangen eines Ultraschallstrahls,
wobei die geänderte Bedingung zum Senden und Empfangen eines Ultraschallstrahls (421) einen Steuerwinkel eines Ultraschallstrahls (421) enthält, und wobei das Bild des interessierenden Objekts (410), das auf der geänderten Bedingung zum Senden und Empfangen eines Ultraschallstrahls (421) basiert, und das erste Ultraschallbild als Bilder des Objekts, das in unterschiedlichen Winkeln betrachtet wird, ausgebildet ist.

11. Verfahren nach Anspruch 10, wobei das Detektieren der zweiten Position auf einem Grad der Korrelation zwischen wenigstens einem von Pixelwerten und Voxelwerten des interessierenden Objekts (410) auf den ersten Ultraschalldaten und wenigstens einem von Pixelwerten und Voxelwerten auf den zweiten Ultraschalldaten basiert.

12. Verfahren nach Anspruch 10, wobei beim Ändern der Bedingung zum Senden und Empfangen eines Ultraschallstrahls (421) die Bedingung zum Senden und Empfangen eines Ultraschallstrahls (421) des Weiteren basierend auf der ersten Position geändert wird,
wobei das Detektieren der ersten Position das Erlangen eines ersten Koordinatenwerts, der die erste Position auf den ersten Ultraschalldaten anzeigt, das Detektieren der zweiten Position, das Erlangen eines zweiten Koordinatenwerts, der die zweite Position auf den zweiten Ultraschalldaten anzeigt, umfasst, und das Ändern der Bedingung zum Senden und Empfangen eines Ultraschallstrahls (421), der in Richtung des Objekts (400) übertragen wird, das Ändern der Bedingung zum Senden und Empfangen eines Ultraschallstrahls (421) basierend auf einem Differenzwert zwischen dem ersten Koordinatenwert und dem zweiten Koordinatenwert umfasst, und
wobei das Detektieren der ersten Position das Erlangen eines Koordinatenwerts eines Mittelpunkts des interessierenden Objekts (410) auf den ersten Ultraschalldaten als den ersten Koordinatenwert umfasst, und das Detektieren der zweiten Position das Erlangen eines Koordinatenwerts eines Mittelpunkts des interessierenden Objekts (410) auf den zweiten Ultraschalldaten als den zweiten Koordinatenwert umfasst.

13. Verfahren nach Anspruch 10, wobei das Ändern der Bedingung zum Senden und Empfangen eines Ultraschallstrahls (421), der in Richtung des Objekts (400) übertragen wird, das Ändern der Bedingung zum Senden und Empfangen eines Ultraschallstrahls (421) basierend auf einem Differenzwert zwischen der zweiten Position und einer voreingestellten Position umfasst.

14. Nicht flüchtiges, computerlesbares Speichermedium, auf dem ein Programm gespeichert ist, das, wenn es auf einer Ultraschalldiagnosevorrichtung ausgeführt wird, das in einem der Ansprüche 10 bis 13 definierte Verfahren durchführt.

## Revendications

1. Appareil de diagnostic ultrasonique (300) comprenant :
un acquéreur de données (310) configuré pour acquérir des premières données ultrasoniques et des deuxièmes données ultrasoniques suivantes par rapport à un objet (400) ;
un contrôleur (320) configuré pour détecter une première position d'un objet d'intérêt (410) inclus dans l'objet (400) sur les premières données ultrasoniques, détecter une deuxième position de l'objet d'intérêt (410) sur la base de la première position sur les deuxièmes données ultrasoniques,
changer une condition pour émettre un faisceau ultrasonique (421) sur la base de la deuxième position, et contrôler pour acquérir une image de l'objet d'intérêt sur la base de la condition modifiée pour émettre un faisceau ultrasonique (421); et
un écran configuré pour afficher l'image acquise de l'objet d'intérêt sur la base de la condition modifiée pour émettre en changeant au moins l'une de la forme, de la taille et de la position d'une première image ultrasonique incluant l'objet d'intérêt (410) sur la base des premières données ultrasoniques en fonction de la condition modifiée pour émettre un faisceau ultrasonique,
dans lequel la condition modifiée pour émettre un faisceau ultrasonique (421) comprend un angle de braquage d'un faisceau ultrasonique (421), l'image de l'objet d'intérêt (410) sur la base de la condition modifiée pour émettre un faisceau ultrasonique (421) et la première image ultrasonique étant des images de l'objet vu sous différents angles.

2. Appareil de diagnostic ultrasonique (300) selon la revendication 1, dans lequel le contrôleur (320) est configuré pour détecter la seconde position de l'objet d'intérêt (410) sur les deuxièmes données ultrasoniques sur la base d'un degré de corrélation entre au moins une des valeurs de pixel et des valeurs de voxel de l'objet d'intérêt (410) sur les premières données ultrasoniques et au moins une des valeurs de pixels et des valeurs de voxel sur les deuxièmes données ultrasoniques.

3. Appareil de diagnostic ultrasonique (300) selon la revendication 1, dans lequel le contrôleur (320) est configuré pour modifier la condition d'émission d'un faisceau ultrasonique (421) en fonction de la première position.

4. Appareil de diagnostic ultrasonique (300) selon la revendication 3, dans lequel le contrôleur (320) est configuré pour acquérir une première valeur de coordonnée indiquant la première position sur les premières données ultrasoniques, acquérir une deuxième valeur de coordonnée indiquant la deuxième position sur les deuxièmes données ultrasoniques, et changer la condition pour émettre un faisceau ultrasonique (421) basé sur une valeur de différence entre la première valeur de coordonnée et la deuxième valeur de coordonnée.

5. Appareil de diagnostic ultrasonique (300) selon la revendication 4, dans lequel le contrôleur (320) est configuré pour acquérir une valeur de coordonnée d'un point central de l'objet d'intérêt (410) sur les premières données ultrasoniques, comme première valeur de coordonnée, et une valeur de coordonnée d'un point central de l'objet d'intérêt (410) sur les secondes données ultrasoniques, comme deuxième valeur de coordonnée.

6. Appareil de diagnostic ultrasonique (300) selon la revendication 1, dans lequel le contrôleur (320) est configuré pour changer la condition pour émettre un faisceau ultrasonique (421) sur la base d'une valeur de différence entre la deuxième position et une position prédéfinie.

7. Appareil de diagnostic ultrasonique (300) selon la revendication 1, comprenant en outre une unité d'entrée configurée pour recevoir une entrée d'utilisateur pour établir une région d'intérêt (ROI) sur la première image ultrasonique sur la base des premières données ultrasoniques, dans lequel le contrôleur (320) est configuré pour détecter la première position de l'objet d'intérêt (410) dans la ROI.

8. Appareil de diagnostic ultrasonique (300) selon la revendication 1, dans lequel la condition pour émettre un faisceau ultrasonique (421) comprend au moins l'une d'une profondeur de réception d'un faisceau ultrasonique (421), d'une largeur d'un faisceau ultrasonique (421) et d'une position de focalisation d'un faisceau ultrasonique (421).

9. Appareil de diagnostic ultrasonique (300) selon la revendication 1, dans lequel le contrôleur (320) est configuré pour changer en temps réel la condition pour émettre un faisceau ultrasonique (421) transmis vers l'objet (400).

10. Procédé de fonctionnement d'un appareil de diagnostic ultrasonique (300), le procédé comprenant :
l'acquisition de premières données ultrasoniques par rapport à un objet (400) comprenant un objet d'intérêt (410);
la détection d'une première position de l'objet d'intérêt (410) sur les premières données ultrasoniques;
l'acquisition de deuxièmes données ultrasoniques par rapport à l'objet (400) ;
la détection d'une seconde position de l'objet d'intérêt (410) sur la base de la première position sur les deuxièmes données ultrasoniques;
la modification d'une condition pour émettre un faisceau ultrasonique (421) transmis vers l'objet (400) sur la base de la deuxième position;
l'acquisition d'une image de l'objet d'intérêt (410) sur la base de la condition modifiée pour émettre un faisceau d'ultrasons (421); et
l'affichage de l'image acquise de l'objet d'intérêt sur la base de la condition modifiée pour émettre en changeant au moins l'une de la forme, de la taille et de la position d'une première image ultrasonique incluant l'objet d'intérêt sur la base des premières données ultrasoniques en fonction de la condition modifiée pour émettre un faisceau ultrasonique,
dans lequel la condition modifiée pour émettre un faisceau ultrasonique (421) comprend un angle de braquage d'un faisceau ultrasonique (421), l'image de l'objet d'intérêt (410) sur la base de la condition modifiée pour émettre un faisceau ultrasonique (421) et la première image ultrasonique étant des images de l'objet vu sous différents angles.

11. Procédé selon la revendication 10, dans lequel la détection de la deuxième position est basée sur un degré de corrélation entre au moins une des valeurs de pixel et des valeurs de voxel de l'objet d'intérêt (410) sur les premières données ultrasoniques et au moins une des valeurs de pixel et des valeurs de voxel sur les deuxièmes données ultrasoniques.

12. Procédé selon la revendication 10, dans lequel, dans le changement de la condition pour émettre un faisceau ultrasonique (421),
la condition pour
émettre un faisceau ultrasonique (421) est modifiée en outre sur la base de la première position,
dans lequel la détection de la première position comprend l'acquisition d'une première valeur de coordonnée indiquant la première position sur les premières données ultrasoniques, la détection de la deuxième position comprend l'acquisition d'une deuxième valeur de coordonnée indiquant la deuxième position sur les deuxièmes données ultrasoniques, et le changement de la condition pour émettre un faisceau ultrasonique (421) transmis vers l'objet (400) comprend le changement de la condition pour émettre un faisceau ultrasonique (421) basé sur une valeur de différence entre la première valeur de coordonnée et la deuxième valeur de coordonnée, et
dans lequel la détection de la première position comprend l'acquisition d'une valeur de coordonnée d'un point central de l'objet d'intérêt (410) sur les premières données ultrasoniques, comme première valeur de coordonnée, et la détection de la deuxième position comprend une valeur de coordonnée d'un point central de l'objet d'intérêt (410) sur les secondes données ultrasoniques, comme deuxième valeur de coordonnée.

13. Procédé selon la revendication 10, dans lequel le changement de la condition pour émettre un faisceau ultrasonique (421) transmis vers l'objet (400) comprend le changement de la condition pour émettre un faisceau ultrasonique (421) sur la base d'une valeur de différence entre la seconde position et une position prédéfinie.

14. Support de stockage lisible par ordinateur non transitoire sur lequel est stocké un programme qui, lorsqu'il est exécuté sur un appareil de diagnostic ultrasonique, exécute le procédé défini dans l'une quelconque des revendications 10-13.
